(19) Europäisches Patentamt / European Patent Office / Office européen des brevets

(11) **EP 4 696 719 A1**

(12) **EUROPEAN PATENT APPLICATION**
published in accordance with Art. 153(4) EPC

(43) Date of publication:
**18.02.2026 Bulletin 2026/08**

(21) Application number: **24788775.5**

(22) Date of filing: **10.04.2024**

(51) International Patent Classification (IPC):
*C08F 2/20* (2006.01)  *C08F 20/06* (2006.01)
*C08J 3/24* (2006.01)  *C08L 33/02* (2006.01)

(52) Cooperative Patent Classification (CPC):
**C08F 2/20; C08F 20/06; C08J 3/24; C08L 33/02**

(86) International application number:
**PCT/JP2024/014596**

(87) International publication number:
**WO 2024/214752 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.04.2023 JP 2023065971**

(71) Applicant: **SUMITOMO SEIKA Chemicals Co., Ltd.
Kako-gun, Hyogo 675-0145 (JP)**

(72) Inventor: **IIJIMA, Midori
Himeji-shi, Hyogo 672-8076 (JP)**

(74) Representative: **Müller-Boré & Partner
Patentanwälte PartG mbB
Friedenheimer Brücke 21
80639 München (DE)**

(54) **METHOD FOR PRODUCING WATER-ABSORBING RESIN PARTICLES, WATER-ABSORBING RESIN PARTICLES, ABSORBER, AND ABSORBENT ARTICLE**

(57) There is provided a novel method for producing water-absorbent resin particles, the method being capable of producing water-absorbent resin particles having improved absorption performance (in particular, in terms of physiological saline retention capacity, physiological saline absorption capacity under a load of 4.14 kPa, and value of non-pressurization DW after 5 minutes). A method for producing water-absorbent resin particles by subjecting a water-soluble ethylenically unsaturated monomer to reversed phase suspension polymerization using a radical polymerization initiator, wherein the radical polymerization initiator comprises an azo compound as at least one radical polymerization initiator, wherein the reversed phase suspension polymerization comprises two or more stages of polymerization steps, wherein in a first-stage polymerization step, (a) an internal-crosslinking agent is used in an amount of 0.01 mmol or more and 0.11 mmol or less per mole of the water-soluble ethylenically unsaturated monomer used in first-stage polymerization, wherein in a second-stage polymerization step, (b) an internal-crosslinking agent is used in an amount of 0 mmol or more and 0.042 mmol or less per mole of the water-soluble ethylenically unsaturated monomer used in second-stage polymerization, and (c) the azo compound is used in an amount of 0.38 mmol or less per mole of the water-soluble ethylenically unsaturated monomer used in the second-stage polymerization, and wherein the method comprises a surface-crosslinking step after the second- or subsequent-stage polymerization step.

EP 4 696 719 A1

**Description**

Technical Field

The present invention relates to a method for producing water-absorbent resin particles, water-absorbent resin particles, an absorbent material, and an absorbent article; and more particularly to a method for producing a water-absorbent resin constituting an absorbent material suitably used for hygienic materials, such as disposable diapers, sanitary napkins, and incontinence pads, water-absorbent resin particles, an absorbent material obtained using the water-absorbent resin particles, and an absorbent article.

Background Art

In recent years, water-absorbent resins have been widely used in the field of hygienic materials, such as disposable diapers, sanitary napkins, and incontinence pads.

As such water-absorbent resin particles, crosslinked products of polymers of a water-soluble ethylenically unsaturated monomer, more specifically, crosslinked products of polymers of partially neutralized polyacrylic acid, have been proposed as preferable water-absorbent resin particles, because of many advantages: for example, they have good water absorption capacity; acrylic acid used as a raw material is readily industrially available, and thus, they can be produced at low cost with uniform quality; additionally, they are resistant to decomposition or degradation (see, for example, Patent Literature 1).

An absorbent article, such as a disposable diaper, a sanitary napkin, or an incontinence pad, is composed of an absorbent material that absorbs and retains a body liquid, such as urine or menses excreted from the body, the absorbent material being positioned mainly in a central portion, a liquid-permeable front sheet (top sheet) positioned on the side of the absorbent article that is brought into contact with the body, and a liquid-impermeable rear sheet (back sheet) positioned opposite to the side that is brought into contact with the body. The absorbent material is typically composed of hydrophilic fibers, such as pulp, and water-absorbent resin particles.

Citation List

Patent Literature

Patent Literature 1: JP-A-H3-227301

Summary of Invention

Technical Problem

In such an absorbent article, the water-absorbent resin particles contained in the absorbent material are required to have a higher water retention capacity. Using the water-absorbent resin particles with a high water retention capacity in the absorbent article can, for example, reduce the amount of the water-absorbent resin particles and the amount of hydrophilic fibers such as pulp used in the absorbent article, in view of maintaining the water retention capacity of the entire absorbent article; however, it may reduce other performance of the absorbent article (for example, in terms of permeation rate, re-wet, or liquid leakage). Thus, improving the absorption performance of the water-absorbent resin particles can reduce the amount of the water-absorbent resin particles or the amount of hydrophilic fibers such as pulp used in the absorbent article, while maintaining the performance of the absorbent article.

The inventor of the present invention focused on the physiological saline retention capacity, the physiological saline absorption capacity under a load of 4.14 kPa, and the value of non-pressurization Demand Wettability (hereinafter, non-pressurization DW) after 5 minutes, in view of improving the absorption performance of the water-absorbent resin particles used in the absorbent article.

Under such circumstances, it is a main object of the present invention to provide a novel method for producing water-absorbent resin particles, the method being capable of producing water-absorbent resin particles having improved absorption performance (in particular, in terms of physiological saline retention capacity, physiological saline absorption capacity under a load of 4.14 kPa, and value of non-pressurization DW after 5 minutes). It is also an object of the present invention to provide water-absorbent resin particles having improved absorption performance (in particular, in terms of physiological saline retention capacity, physiological saline absorption capacity under a load of 4.14 kPa, and value of non-pressurization DW after 5 minutes), an absorbent material obtained using the water-absorbent resin particles, and an absorbent article.

Solution to Problem

The present inventor has conducted extensive research to solve the aforementioned problem. As a result, the present inventor has found that water-absorbent resin particles having improved absorption performance (in particular, in terms of physiological saline retention capacity, physiological saline absorption capacity under a load of 4.14 kPa, and value of non-pressurization DW after 5 minutes) can be produced using a method for producing water-absorbent resin particles by subjecting a water-soluble ethylenically unsaturated monomer to reversed phase suspension polymerization using a radical polymerization initiator, in which an azo compound is used as the radical polymerization initiator; the reversed phase suspension polymerization is performed in two or more stages; the amount of the internal-crosslinking agent used in first-stage polymerization, the amount of the internal-crosslinking agent used in second-stage polymerization, and the amount of the azo compound used in the second-stage polymerization are each adjusted within a respective predetermined range; and a surface-crosslinking step is included after the polymerization. The present invention has been accomplished as a result of further research based on these findings.

In summary, the present invention provides aspects of the invention comprising the following features:

Item 1. A method for producing water-absorbent resin particles by subjecting a water-soluble ethylenically unsaturated monomer to reversed phase suspension polymerization using a radical polymerization initiator,

wherein the radical polymerization initiator comprises an azo compound as at least one radical polymerization initiator,
wherein the reversed phase suspension polymerization comprises two or more stages of polymerization steps,
wherein in a first-stage polymerization step,

(a) an internal-crosslinking agent is used in an amount of 0.01 mmol or more and 0.11 mmol or less per mole of the water-soluble ethylenically unsaturated monomer used in first-stage polymerization,
wherein in a second-stage polymerization step,
(b) an internal-crosslinking agent is used in an amount of 0 mmol or more and 0.042 mmol or less per mole of the water-soluble ethylenically unsaturated monomer used in second-stage polymerization, and
(c) the azo compound is used in an amount of 0.38 mmol or less per mole of the water-soluble ethylenically unsaturated monomer used in the second-stage polymerization, and

wherein the method comprises a surface-crosslinking step after the second- or subsequent-stage polymerization step.

Item 2. The method according to item 1, wherein primary particles obtained in the first-stage polymerization step, when prepared as a gel swollen 40-fold with ion exchange water, have a storage elastic modulus of 10 Pa or more and 200 Pa or less, and
wherein secondary particles obtained in the second- or subsequent-stage polymerization step, when prepared as a gel swollen 40-fold with ion exchange water, have a storage elastic modulus of 5 Pa or more and 200 Pa or less.

Item 3. The method according to item 1 or 2, wherein the water-absorbent resin particles have the following properties (A) to (C):

(A) a physiological saline retention capacity of 50 g/g or more and 80 g/g or less,
(B) a physiological saline absorption capacity under a load of 4.14 kPa of 15 g/g or more and 40 g/g or less, and
(C) a value of non-pressurization DW after 5 minutes of 50 mL/g or more and 80 mL/g or less.

Item 4. Water-absorbent resin particles, the water-absorbent resin particles being a crosslinked product of a polymer of a water-soluble ethylenically unsaturated monomer,
wherein the water-absorbent resin particles have the following properties (A) to (C):

(A) a physiological saline retention capacity of 50 g/g or more and 80 g/g or less;
(B) a physiological saline absorption capacity under a load of 4.14 kPa of 15 g/g or more and 40 g/g or less; and
(C) a value of non-pressurization DW after 5 minutes of 50 mL/g or more and 80 mL/g or less.

Item 5. An absorbent material comprising the water-absorbent resin particles according to item 4.
Item 6. An absorbent article comprising the absorbent material according to item 5.

Advantageous Effects of Invention

According to the present invention, it is possible to provide a novel method for producing water-absorbent resin particles, the method being capable of producing water-absorbent resin particles having improved absorption performance (in particular, in terms of physiological saline retention capacity, physiological saline absorption capacity under a load of 4.14 kPa, and value of non-pressurization DW after 5 minutes). Furthermore, according to the present invention, it is also possible to provide water-absorbent resin particles having better absorption performance (in particular, in terms of physiological saline retention capacity, physiological saline absorption capacity under a load of 4.14 kPa, and value of non-pressurization DW after 5 minutes), an absorbent material obtained using the water-absorbent resin particles, and an absorbent article.

Brief Description of Drawings

Fig. 1 is a schematic diagram of an apparatus for measuring the non-pressurization DW of the water-absorbent resin particles.
Fig. 2 is a schematic diagram of an apparatus for measuring the physiological saline absorption capacity under a load of 4.14 kPa of the water-absorbent resin particles.

Description of Embodiments

As used herein, the term "comprising" includes "consisting essentially of" and "consisting of". As used herein, the term "(meth)acrylic" refers to "acrylic or methacrylic", the term "(meth)acrylate" refers to "acrylate or methacrylate", and the term "(poly)" means both cases with and without the prefix "poly". As used herein, the term "water-soluble" refers to having a water solubility of 5% by mass or more at 25°C.
As used herein, values connected with "to" refer to the numerical range including the values before and after "to" as the lower and upper limits. When a plurality of lower limits and a plurality of upper limits are mentioned separately, any lower limit and any upper limit may be selected and connected with "to".

1. Method for Producing Water-Absorbent Resin Particles

The method for producing water-absorbent resin particles of the present invention is a method for producing water-absorbent resin particles by subjecting a water-soluble ethylenically unsaturated monomer to reversed phase suspension polymerization using a radical polymerization initiator. The radical polymerization initiator comprises an azo compound as at least one radical polymerization initiator. In the method for producing water-absorbent resin particles of the present invention, the reversed phase suspension polymerization comprises two or more stages of polymerization steps.
In the method for producing water-absorbent resin particles of the present invention, in a first-stage polymerization step of the reversed phase suspension polymerization, (a) an internal-crosslinking agent is used in an amount of 0.01 mmol or more and 0.11 mmol or less per mole of the water-soluble ethylenically unsaturated monomer used in first-stage polymerization. Moreover, in a second-stage polymerization step, (b) an internal-crosslinking agent is used in an amount of 0 mmol or more and 0.042 mmol or less per mole of the water-soluble ethylenically unsaturated monomer used in second-stage polymerization, and (c) the azo compound is used in an amount of 0.38 mmol or less per mole of the water-soluble ethylenically unsaturated monomer used in the second-stage polymerization. Furthermore, the method for producing water-absorbent resin particles of the present invention comprises a surface-crosslinking step after the second- or subsequent-stage polymerization step.
Because of these features, the method for producing water-absorbent resin particles of the present invention can satisfactorily produce water-absorbent resin particles having better absorption performance (in particular, in terms of physiological saline retention capacity, physiological saline absorption capacity under a load of 4.14 kPa, and value of non-pressurization DW after 5 minutes). The method for producing water-absorbent resin particles of present invention will be hereinafter described in detail.

[Reversed Phase Suspension Polymerization]

Reversed phase suspension polymerization involves dispersing an aqueous monomer solution containing a water-soluble ethylenically unsaturated monomer in a dispersion medium, in the presence of a dispersion stabilizer, for example. As described later, the dispersion medium is preferably a hydrocarbon dispersion medium, for example.

Conventionally, when a water-soluble ethylenically unsaturated monomer is subjected to reversed phase suspension polymerization to produce water-absorbent resin particles, the reversed phase suspension polymerization may be performed in only a single-stage polymerization step. However, in the reversed phase suspension polymerization according to the present invention, the polymerization step is performed in two or more multiple stages. The reversed phase suspension polymerization is preferably performed in two or three stages of polymerization steps, in view of enhancing the productivity of the water-absorbent resin particles while satisfactorily achieving the effects of the present invention.

The reversed phase suspension polymerization may be performed as follows: the first-stage polymerization step is performed (first stage of reversed phase suspension polymerization); subsequently, a water-soluble ethylenically unsaturated monomer is added to and mixed with the reaction mixture obtained by the first-stage polymerization reaction, and the second- or subsequent-stage polymerization step is performed in the same manner as in the first-stage polymerization step (first stage of reversed phase suspension polymerization). In each of the second- and subsequent-stages of reversed phase suspension polymerization, reversed phase suspension polymerization is preferably performed by adding, in addition to the water-soluble ethylenically unsaturated monomer, a predetermined amount of a polymerization initiator, based on the amount of the water-soluble ethylenically unsaturated monomer added in each of the second- and subsequent-stages of reversed phase suspension polymerization. In the second- or subsequent-stage polymerization, an internal-crosslinking agent may also be optionally added to the water-soluble ethylenically unsaturated monomer.

As described later, a dispersion stabilizer (a surfactant and a polymeric dispersion agent) is also preferably used in the reversed phase suspension polymerization. As long as the dispersion stabilizer is added before the beginning of the polymerization reaction, it may be added either before or after addition of the aqueous monomer solution.

In view of readily reducing the amount of the hydrocarbon dispersion medium remaining in the resulting water-absorbent resin particles, it is preferred to disperse the aqueous monomer solution in the hydrocarbon dispersion medium in which a polymeric dispersion agent is dispersed, and then disperse a surfactant in the resulting dispersion, followed by polymerization.

The reaction temperature during the polymerization reaction is preferably 20 to 110°C, and more preferably 40 to 90°C, in view of allowing the polymerization to proceed quickly to reduce the polymerization time for improved economic efficiency, and readily removing the heat of polymerization to perform the reaction smoothly.

<Polymerization Steps>

In each of the first-stage and second- and subsequent-stages of polymerization steps, the polymerization reaction of a water-soluble ethylenically unsaturated monomer is performed by reversed phase suspension polymerization using a radical polymerization initiator. Specifically, an aqueous solution containing a water-soluble ethylenically unsaturated monomer, a hydrocarbon dispersion medium, a radical polymerization initiator, and an internal-crosslinking agent are mixed, and the polymerization reaction of the water-soluble ethylenically unsaturated monomer is allowed to proceed.

In the present invention, in the first-stage polymerization step, (a) an internal-crosslinking agent is used in an amount of 0.01 mmol or more and 0.11 mmol or less per mole of the water-soluble ethylenically unsaturated monomer used in first-stage polymerization. Furthermore, in the present invention, in the second-stage polymerization step, (b) an internal-crosslinking agent is used in an amount of 0 mmol or more and 0.042 mmol or less per mole of the water-soluble ethylenically unsaturated monomer used in second-stage polymerization, and (c) the azo compound is used in an amount of 0.38 mmol or less per mole of the water-soluble ethylenically unsaturated monomer used in the second-stage polymerization.

Each of the first-stage and second- and subsequent-stages of polymerization steps will be hereinafter described in detail.

[Water-Soluble Ethylenically Unsaturated Monomer]

Examples of the water-soluble ethylenically unsaturated monomer include (meth)acrylic acid and salts thereof; 2-(meth)acrylamido-2-methylpropanesulfonic acid and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers, such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethyl(meth)acrylamide, and more preferred are (meth)acrylic acid and salts thereof, because they are readily industrially available, for example. These water-soluble ethylenically unsaturated monomers may be used alone or in combinations of two or more.

Among these water-soluble ethylenically unsaturated monomers, acrylic acid and salts thereof are widely used as raw materials of water-absorbent resin particles. Copolymers of acrylic acid and/or salts thereof with other water-soluble ethylenically unsaturated monomers as mentioned above may also be used. In the present invention, the water-soluble ethylenically unsaturated monomer contains 70 to 100 mol% of acrylic acid and a salt thereof. That is, the content of acrylic acid and a salt thereof in the total water-soluble ethylenically unsaturated monomers is 70 to 100 mol%.

The water-soluble ethylenically unsaturated monomer as an aqueous solution is dispersed in a hydrocarbon dispersion medium and then subjected to reversed phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturation concentration.

The concentration of the water-soluble ethylenically unsaturated monomer in the first-stage polymerization is more preferably 55% by mass or less, still more preferably 50% by mass or less, and even more preferably 45% by mass or less, in view of improving the physiological saline retention capacity, the physiological saline absorption capacity under a load of 4.14 kPa, and the value of non-pressurization DW after 5 minutes. On the other hand, the concentration of the water-soluble ethylenically unsaturated monomer is preferably 25% by mass or more, more preferably 30% by mass or more, and still more preferably 35% by mass or more, in view of improving the physiological saline retention capacity, the physiological saline absorption capacity under a load of 4.14 kPa, and the value of non-pressurization DW after 5 minutes.

The concentration of the water-soluble ethylenically unsaturated monomer in the second-stage polymerization is more preferably 55% by mass or less, still more preferably 50% by mass or less, and even more preferably 45% by mass or less, in view of improving the physiological saline retention capacity, the physiological saline absorption capacity under a load of 4.14 kPa, and the value of non-pressurization DW after 5 minutes. On the other hand, the concentration of the water-soluble ethylenically unsaturated monomer is preferably 30% by mass or more, more preferably 35% by mass or more, and still more preferably 40% by mass or more, in view of improving the physiological saline retention capacity, the physiological saline absorption capacity under a load of 4.14 kPa, and the value of non-pressurization DW after 5 minutes.

The ratio of the amount of the water-soluble ethylenically unsaturated monomer used in the second-stage polymerization to the amount of the water-soluble ethylenically unsaturated monomer used in the first-stage polymerization (the amount of the water-soluble ethylenically unsaturated monomer used in the second-stage polymerization/the amount of the water-soluble ethylenically unsaturated monomer used in the first-stage polymerization) is preferably 0.1 to 3.0, more preferably 0.5 to 2.5, still more preferably 1.0 to 2.0, and even more preferably 1.3 to 1.5, in view of improving the physiological saline retention capacity, the physiological saline absorption capacity under a load of 4.14 kPa, and the value of non-pressurization DW after 5 minutes.

In the water-soluble ethylenically unsaturated monomer to be used, an acid group (acid group of acrylic acid) may be optionally neutralized beforehand with an alkaline neutralizing agent. Examples of such alkaline neutralizing agents include alkali metal salts, such as sodium hydroxide, sodium carbonate, sodium hydrogen carbonate, potassium hydroxide, and potassium carbonate; and ammonia. These alkaline neutralizing agents may be used in the form of an aqueous solution to facilitate the neutralization process. The above-mentioned alkaline neutralizing agents may be used alone or in combinations of two or more.

In view of improving the physiological saline retention capacity, the physiological saline absorption capacity under a load of 4.14 kPa, and the value of non-pressurization DW after 5 minutes, the neutralization degree of the water-soluble ethylenically unsaturated monomer with an alkaline neutralizing agent, calculated as the neutralization degree of all acid groups in the water-soluble ethylenically unsaturated monomer, is preferably 10 to 100 mol%, more preferably 30 to 90 mol%, still more preferably 50 to 85 mol%, and even more preferably 70 to 80 mol%.

[Polymerization Initiator]

The polymerization initiator to be mixed in the polymerization steps is a radical polymerization initiator, and the radical polymerization initiator comprises an azo compound as at least one radical polymerization initiator.

The azo compound can be used alone as the radical polymerization initiator. In view of improving the physiological saline retention capacity, the physiological saline absorption capacity under a load of 4.14 kPa, and the value of non-pressurization DW after 5 minutes, the azo compound is preferably used in combination with a peroxide.

The use of the azo compound in combination with a peroxide does not necessarily require that the azo compound and the peroxide be present together at the beginning of the polymerization reaction, but refers to the state in which one of the compounds is present while the monomer conversion due to the radical cleavage of another compound is less than 10%. Preferably, both compounds are present together in the aqueous solution containing the water-soluble ethylenically unsaturated monomer before the beginning of the polymerization reaction. The azo compound and the peroxide may be added to the polymerization reaction system through separate flow passages or may be sequentially added to the polymerization reaction system through the same flow passage.

The azo compound and the peroxide to be used may each be in the form of a powder or an aqueous solution.

Examples of the peroxide include persulfates, such as potassium persulfate, ammonium persulfate, and sodium persulfate; and peroxides, such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide. Among these peroxides, potassium persulfate, ammonium persulfate, and sodium persulfate are preferred because they are readily available and easy to handle.

In view of improving the physiological saline retention capacity, the physiological saline absorption capacity under a load of 4.14 kPa, and the value of non-pressurization DW after 5 minutes, the amount of the peroxide used in the first-stage polymerization is preferably 0.001 to 1.0 mmol, more preferably 0.01 to 0.50 mmol, still more preferably 0.05 to 0.30 mmol, and even more preferably 0.075 to 0.15 mmol, per mole of the water-soluble ethylenically unsaturated monomer used in the first-stage polymerization.

In view of improving the physiological saline retention capacity, the physiological saline absorption capacity under a load of 4.14 kPa, and the value of non-pressurization DW after 5 minutes, the amount of the peroxide used in the second-stage polymerization is preferably 0.001 to 1.0 mmol, more preferably 0.01 to 0.50 mmol, still more preferably 0.05 to 0.20 mmol, and even more preferably 0.075 to 0.15 mmol, per mole of the water-soluble ethylenically unsaturated monomer used in the second-stage polymerization.

Examples of the azo compound include 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Among these azo compounds, 2,2'-azobis(2-amidinopropane) dihydrochloride is preferred because it is readily available and easy to handle.

In view of improving the physiological saline retention capacity, the physiological saline absorption capacity under a load of 4.14 kPa, and the value of non-pressurization DW after 5 minutes, the amount of the azo compound used in the first-stage polymerization is preferably 0 to 1.0 mmol, more preferably 0 to 0.50 mmol, still more preferably 0.10 to 0.38 mmol, and even more preferably 0.20 to 0.35 mmol, per mole of the water-soluble ethylenically unsaturated monomer used in the first-stage polymerization.

In the method of the present invention, (c) the azo compound is used in an amount of 0.38 mmol or less per mole of the water-soluble ethylenically unsaturated monomer used in the second-stage polymerization. In view of improving the physiological saline retention capacity, the physiological saline absorption capacity under a load of 4.14 kPa, and the value of non-pressurization DW after 5 minutes, the amount of the azo compound used in the second-stage polymerization is preferably 0 to 0.35 mmol, more preferably 0.05 to 0.35 mmol, and still more preferably 0.10 to 0.35 mmol, per mole of the water-soluble ethylenically unsaturated monomer used in the second-stage polymerization.

The polymerization initiator may also be used in combination with a reducing agent, such as sodium sulfite, sodium hydrogensulfite, ferrous sulfate, or L-ascorbic acid, and used as a redox polymerization initiator.

In view of improving the physiological saline retention capacity, the physiological saline absorption capacity under a load of 4.14 kPa, and the value of non-pressurization DW after 5 minutes, the molar ratio of the peroxide to the azo compound (peroxide/azo compound) in the first-stage polymerization is preferably in the range of 0.1 to 1.0, more preferably in the range of 0.2 to 0.5, and still more preferably in the range of 0.3 to 0.4.

In view of improving the physiological saline retention capacity, the physiological saline absorption capacity under a load of 4.14 kPa, and the value of non-pressurization DW after 5 minutes, the molar ratio of the peroxide to the azo compound (peroxide/azo compound) in the second-stage polymerization is preferably in the range of 0.1 to 1.5, more preferably in the range of 0.2 to 1.0, and still more preferably in the range of 0.3 to 0.7.

[Internal-Crosslinking Agent]

Examples of the internal-crosslinking agent include those that can crosslink the polymer of the water-soluble ethylenically unsaturated monomer used, for example: unsaturated polyesters obtained by reacting polyols, such as diols and triols, e.g., (poly)ethylene glycol, (poly)propylene glycol, 1,4-butanediol, trimethylolpropane, and (poly)glycerin, with unsaturated acids, such as (meth)acrylic acid, maleic acid, and fumaric acid; bisacrylamides, such as N,N-methylenebisacrylamide; di or tri(meth)acrylic acid esters obtained by reacting polyepoxides with (meth)acrylic acid; carbamyl di(meth)acrylates obtained by reacting polyisocyanates, such as tolylene diisocyanate and hexamethylene diisocyanate, with hydroxyethyl (meth)acrylate; compounds having two or more polymerizable unsaturated groups, such as allylated starch, allylated cellulose, diallyl phthalate, N,N',N''-triallyl isocyanurate, and divinylbenzene; polyglycidyl compounds, such as diglycidyl compounds, e.g., (poly)ethylene glycol diglycidyl ether, (poly)propylene glycol diglycidyl ether, and (poly)glycerin diglycidyl ether, and triglycidyl compounds; epihalohydrin compounds, such as epichlorohydrin, epibromohydrin, and α-methylepichlorohydrin; compounds having two or more reactive functional groups, such as isocyanate compounds, e.g., 2,4-tolylene diisocyanate and hexamethylene diisocyanate; and oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, the unsaturated polyesters or polyglycidyl compounds are preferably used, the diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combinations of two or more.

In the present invention, in the first-stage polymerization step, (a) an internal-crosslinking agent is used in an amount of 0.01 mmol or more and 0.11 mmol or less per mole of the water-soluble ethylenically unsaturated monomer used in first-stage polymerization. In view of improving the physiological saline retention capacity, the physiological saline absorption capacity under a load of 4.14 kPa, and the value of non-pressurization DW after 5 minutes, the amount of the internal-crosslinking agent used in the first-stage polymerization is preferably 0.02 to 0.09 mmol, more preferably 0.03 to 0.075 mmol, and still more preferably 0.04 to 0.06 mmol, per mole of the water-soluble ethylenically unsaturated monomer used in the first-stage polymerization.

Moreover, in the present invention, in the second-stage polymerization step, (b) an internal-crosslinking agent is used in an amount of 0 mmol or more and 0.042 mmol or less per mole of the water-soluble ethylenically unsaturated monomer used in second-stage polymerization. In view of improving the physiological saline retention capacity, the physiological saline absorption capacity under a load of 4.14 kPa, and the value of non-pressurization DW after 5 minutes, the amount of the internal-crosslinking agent used in the second-stage polymerization is preferably 0 to 0.038 mmol, more preferably 0 to 0.032 mmol, still more preferably 0 to 0.030 mmol, and particularly preferably 0 to 0.025 mmol, per mole of the water-soluble ethylenically unsaturated monomer used in the second-stage polymerization.

[Hydrocarbon Dispersion Medium]

Examples of the hydrocarbon dispersion medium include $C_{6-8}$ aliphatic hydrocarbons, such as n-hexane, n-heptane, 2-methylhexane, 3-methylhexane, 2,3-dimethylpentane, 3-ethylpentane, and n-octane; alicyclic hydrocarbons, such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons, such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combinations of two or more. The use of a commercially available mixture of hydrocarbon dispersion media, such as Exxsol Heptane (from Exxon Mobil Corporation; containing 75 to 85% by mass of heptane and its isomeric hydrocarbons), also leads to favorable results.

The amount of the hydrocarbon dispersion medium used is preferably 100 to 1500 parts by mass, more preferably 150 to 1000 parts by mass, still more preferably 200 to 500 parts by mass, and even more preferably 300 to 400 parts by mass, per 100 parts by mass of a first-stage water-soluble ethylenically unsaturated monomer, in view of homogeneously dispersing the water-soluble ethylenically unsaturated monomer, and facilitating control of the polymerization temperature.

[Dispersion Stabilizer]

(Surfactant)

In the reversed phase suspension polymerization, a dispersion stabilizer may be optionally used to improve the dispersion stability of the water-soluble ethylenically unsaturated monomer in the hydrocarbon dispersion medium. A surfactant may be used as such a dispersion stabilizer.

Examples of usable surfactants include sucrose fatty acid esters, polyglycerol fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerol fatty acid esters, and sucrose fatty acid esters are particularly preferably used, in view of dispersion stability of the monomer. These surfactants may be used alone or in combinations of two or more.

The amount of the surfactant used is preferably 0.1 to 30 parts by mass, more preferably 0.3 to 10 parts by mass, still more preferably 0.5 to 1 part by mass, and even more preferably 0.6 to 0.9 parts by mass, per 100 parts by mass of the first-stage water-soluble ethylenically unsaturated monomer.

(Polymeric Dispersion Agent)

A polymeric dispersion agent may be used in combination with the above-described surfactant, as the dispersion stabilizer to be used in the reversed phase suspension polymerization.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, in view of dispersion stability of the monomer. These polymeric dispersion agents may be used alone or in combinations of two or more.

The amount of the polymeric dispersion agent used is preferably 0.1 to 30 parts by mass, more preferably 0.3 to 10 parts by mass, still more preferably 0.5 to 1 part by mass, and even more preferably 0.6 to 0.9 parts by mass, per 100 parts by mass of the first-stage water-soluble ethylenically unsaturated monomer.

[Other Components]

In the method for producing water-absorbent resin particles, other components may be optionally added to the aqueous solution containing the water-soluble ethylenically unsaturated monomer, which is then subjected to the reversed phase suspension polymerization. Various additives such as thickeners and chain transfer agents can be added as the other components.

(Thickener)

For example, a thickener may be added to the aqueous solution containing the water-soluble ethylenically unsaturated monomer, which is then subjected to the reversed phase suspension polymerization. By thus adding a thickener to adjust the viscosity of the aqueous solution, the median particle size obtained by the reversed phase suspension polymerization can be controlled.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. Assuming that the stirring rate during the polymerization is the same, the higher the viscosity of the water-soluble ethylenically unsaturated monomer solution, the larger the primary particles and/or secondary particles of the resulting particles tend to be.

(Chain Transfer Agent)

For example, in view of improving the physiological saline retention capacity, the physiological saline absorption capacity under a load of 4.14 kPa, and the value of non-pressurization DW after 5 minutes, the polymerization of the water-soluble ethylenically unsaturated monomer may be performed in the presence of a chain transfer agent.

Examples of the chain transfer agent include thiols, such as ethanethiol, propanethiol, and dodecanethiol; thiol acids, such as thioglycolic acid, thiomalic acid, dimethyldithiocarbamic acid, diethyldithiocarbamic acid, or salts thereof; secondary alcohols, such as isopropanol; phosphorous acid and phosphorous acid compounds, for example, normal salts of phosphorous acid, such as disodium phosphite, dipotassium phosphite, and diammonium phosphite, and acidic salts of phosphorous acid, such as sodium hydrogen phosphite, potassium hydrogen phosphite, and ammonium hydrogen phosphite; phosphoric acid and phosphoric acid compounds, for example, normal salts of phosphoric acid, such as sodium phosphate, potassium phosphate, and ammonium phosphate, and acidic salts of phosphoric acid, such as sodium dihydrogen phosphate, potassium dihydrogen phosphate, ammonium dihydrogen phosphate, disodium hydrogen phosphate, dipotassium hydrogen phosphate, and diammonium hydrogen phosphate; hypophosphorous acid and hypophosphorous acid compounds, for example, hypophosphorous acid salts, such as sodium hypophosphite, potassium hypophosphite, and ammonium hypophosphite; pyrophosphoric acid, tripolyphosphoric acid, polyphosphoric acid, and salts thereof; trimethyl phosphate and nitrilotrimethylenetriphosphonic acid. These chain transfer agents may be used alone or in combinations of two or more. A hydrate thereof may also be used as the chain transfer agent.

In view of improving the physiological saline retention capacity, the physiological saline absorption capacity under a load of 4.14 kPa, and the value of non-pressurization DW after 5 minutes, the amount of the chain transfer agent used in the second-stage polymerization is preferably 0 to 0.0005 mol, more preferably 0 to 0.0003 mol, still more preferably 0 to 0.0002 mol, and even more preferably 0 to 0.0001 mol, per mole of the water-soluble ethylenically unsaturated monomer.

As described above, the inventor of the present invention focused on the physiological saline retention capacity, the physiological saline absorption capacity under a load of 4.14 kPa, and the value of non-pressurization DW after 5 minutes, in view of improving the absorption performance of the water-absorbent resin particles used in the absorbent article. Increasing the value of non-pressurization DW after 5 minutes requires increasing the surface area of the water-absorbent resin particles. In an attempt to increase the surface area of the water-absorbent resin particles, the present inventor has found that by using a method for producing water-absorbent resin particles by subjecting a water-soluble ethylenically unsaturated monomer to reversed phase suspension polymerization, in which the reversed phase suspension polymerization is performed in two or more stages of polymerization steps, so that the storage elastic modulus of primary particles obtained in a first-stage polymerization step is improved to prevent agglomeration of secondary particles obtained in a second-or subsequent-stage polymerization step, the surface area of the water-absorbent resin particles obtained by subjecting the secondary particles to surface-crosslinking is increased, resulting in an increased value of non-pressurization DW after 5 minutes. Increasing the storage elastic modulus of the primary particles obtained in the first-stage polymerization step requires increasing the degree of crosslinking of the primary particles. Unfortunately, increasing the degree of crosslinking of the primary particles reduces the physiological saline retention capacity and the physiological saline absorption capacity under a load of 4.14 kPa.

In view of the above, in the present invention, the storage elastic modulus of the primary particles obtained in the first-stage polymerization step, when prepared as a gel swollen 40-fold with ion exchange water, is preferably set in the range of 10 Pa or more and 200 Pa or less. In view of improving the value of non-pressurization DW after 5 minutes, the storage elastic modulus is preferably 10 Pa or more, more preferably 15 Pa or more, and still more preferably 20 Pa or more. On the other hand, in view of improving the physiological saline retention capacity and the physiological saline absorption capacity under a load of 4.14 kPa, the storage elastic modulus is preferably 200 Pa or less, more preferably 150 Pa or less, and still more preferably 100 Pa or less. In view of improving the physiological saline retention capacity, the physiological saline absorption capacity under a load of 4.14 kPa, and the value of non-pressurization DW after 5 minutes, the storage elastic modulus is preferably in the range of 15 to 150 Pa, and more preferably in the range of about 20 to 100 Pa, for example. The storage elastic modulus can be adjusted, for example, by adjusting the amount of the internal-crosslinking agent used, the concentration of the water-soluble ethylenically unsaturated monomer, the neutralization degree of the water-soluble ethylenically unsaturated monomer, the amount of the polymerization initiator used, and the like in the first-stage polymerization step. The storage elastic modulus refers to the storage elastic modulus measured in a 25°C environment using a stress-controlled rheometer, for the primary particles in the form of a 40-fold swollen gel, prepared by drying the primary particles obtained in the first-stage polymerization step and then causing the resulting particles to swell 40-fold with ion exchange water. The storage elastic modulus is measured using the specific method described in the Examples.

Furthermore, in the present invention, the storage elastic modulus of the secondary particles obtained in the second- or subsequent-stage polymerization step, when prepared as a gel swollen 40-fold with ion exchange water, is preferably set in the range of 5 Pa or more and 200 Pa or less. In view of improving the physiological saline retention capacity, the physiological saline absorption capacity under a load of 4.14 kPa, and the value of non-pressurization DW after 5 minutes, the storage elastic modulus is preferably 5 Pa or more, more preferably 20 Pa or more, and still more preferably 30 Pa or more, while the storage elastic modulus is preferably 200 Pa or less, more preferably 190 Pa or less, still more preferably 170 Pa or less, even more preferably 150 Pa or less, and particularly preferably 100 Pa or less. The storage elastic modulus is preferably in the range of 20 to 190 Pa, more preferably in the range of 20 to 170 Pa, still more preferably in the range of 30 to 150 Pa, and even more preferably in the range of about 30 to 100 Pa, for example. The storage elastic modulus can be adjusted, for example, by adjusting the amount of the internal-crosslinking agent used, the concentration of the water-soluble ethylenically unsaturated monomer, the ratio of the amount of the water-soluble ethylenically unsaturated monomer used in the second-stage polymerization step to the amount of the water-soluble ethylenically unsaturated monomer used in the first-stage polymerization step, the neutralization degree of the water-soluble ethylenically unsaturated monomer, the amount of the polymerization initiator used, the amount of the chain transfer agent used, and the like in the second- or subsequent-stage polymerization step. The storage elastic modulus refers to the storage elastic modulus measured in a 25°C environment using a stress-controlled rheometer, for the secondary particles in the form of a 40-fold swollen gel, prepared by drying the secondary particles obtained in the second-stage polymerization step and then causing the resulting particles to swell 40-fold with ion exchange water. The storage elastic modulus is measured using the specific method described in the Examples.

In view of more satisfactorily achieving the effects of the present invention, the ratio of the storage elastic modulus of the secondary particles to the storage elastic modulus of the primary particles (the storage elastic modulus of the secondary particles/the storage elastic modulus of the primary particles) is preferably 10 times or less, more preferably 5 times or less, and still more preferably 3 times or less. This ratio is preferably in the range of 0.1 to 5 times, and more preferably in the range of 0.5 to 3 times, for example.

As described above, increasing the value of non-pressurization DW after 5 minutes requires increasing the surface area of the water-absorbent resin particles. In an attempt to increase the surface area of the water-absorbent resin particles, the present inventor has found that by using a method for producing water-absorbent resin particles by subjecting a water-soluble ethylenically unsaturated monomer to reversed phase suspension polymerization, in which the reversed phase suspension polymerization is performed in two or more stages of polymerization steps, so that the storage elastic modulus of primary particles obtained in a first-stage polymerization step is improved to prevent agglomeration of secondary particles obtained in a second-or subsequent-stage polymerization step, the surface area of the water-absorbent resin particles obtained by subjecting the secondary particles to surface-crosslinking is increased, resulting in an increased value of non-pressurization DW after 5 minutes. However, increasing the storage elastic modulus of the primary particles obtained in the first-stage polymerization step requires increasing the degree of crosslinking of the primary particles. Unfortunately, increasing the degree of crosslinking of the primary particles reduces the physiological saline retention capacity and the physiological saline absorption capacity under a load of 4.14 kPa. As opposed to this, in the method for producing water-absorbent resin particles of the present invention, the degree of internal-crosslinking formed in the second- or subsequent-stage polymerization is lowered to reduce the storage elastic modulus of the secondary particles before being subjected to surface-crosslinking, and increase the contribution of surface-crosslinking, and this can be assumed to be the reason why water-absorbent resin particles better in physiological saline retention capacity, physiological saline absorption capacity under a load of 4.14 kPa, and value of non-pressurization DW after 5 minutes can be obtained.

<Surface-Crosslinking (Post-Crosslinking) Step>

The surface-crosslinking step is the step of subjecting the secondary particles obtained by polymerizing the water-soluble ethylenically unsaturated monomer in the second- or subsequent-stage polymerization step of reversed phase suspension polymerization (a hydrous gel having an internal-crosslinked structure) to surface-crosslinking. The water-absorbent resin particles of the present invention are obtained by mixing the secondary particles obtained in the polymerization step with a surface-crosslinking agent to crosslink the secondary particles (surface-crosslinking reaction). This surface-crosslinking reaction is preferably performed in the presence of a surface-crosslinking agent after the polymerization of the water-soluble ethylenically unsaturated monomer. In the present invention, the primary particles and secondary particles are prepared, and then the secondary particles are subjected to the surface-crosslinking reaction to give water-absorbent resin particles with improved performance in terms of physiological saline retention capacity, physiological saline absorption capacity under a load of 4.14 kPa, value of non-pressurization DW after 5 minutes, and the like.

In view of improving the physiological saline retention capacity, the physiological saline absorption capacity under a load of 4.14 kPa, and the value of non-pressurization DW after 5 minutes, the molar ratio of the surface-crosslinking agent to the water-soluble ethylenically unsaturated monomer (surface-crosslinking agent/water-soluble ethylenically unsaturated monomer) in the surface-crosslinking step is preferably in the range of 0.00001 to 0.001, more preferably in the range of 0.00005 to 0.00080, still more preferably in the range of 0.00010 to 0.00060, and even more preferably in the range of 0.00020 to 0.00040.

Examples of the surface-crosslinking agent include compounds with two or more reactive functional groups. Examples include polyols, such as ethylene glycol, propylene glycol, 1,4-butanediol, trimethylolpropane, glycerin, polyoxyethylene glycol, polyoxypropylene glycol, and polyglycerin; polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds, such as epichlorohydrin, epibromohydrin, and $\alpha$-methyl epichlorohydrin; isocyanate compounds, such as 2,4-tolylene diisocyanate and hexamethylene diisocyanate; oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds, such as 1,2-ethylenebisoxazoline; carbonate compounds such as ethylene carbonate; and hydroxyalkylamide compounds, such as bis[N,N-di($\beta$-hydroxyethyl)]adipamide. Preferred among these surface-crosslinking agents are polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. These surface-crosslinking agents may be used alone or in combinations of two or more.

The surface-crosslinking agent may be added as is or as an aqueous solution. Optionally, a solution of the surface-crosslinking agent in a hydrophilic organic solvent may be added. Examples of the hydrophilic organic solvent include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; and sulfoxides, such as dimethylsulfoxide. These hydrophilic organic solvents may be used alone, in combinations of two or more, or as a solvent mixture with water.

The surface-crosslinking agent may be added at any time after the polymerization reaction of the water-soluble ethylenically unsaturated monomer is substantially completed. The surface-crosslinking agent is preferably added in the presence of 1 to 400 parts by mass of water, more preferably 5 to 100 parts by mass of water, still more preferably 10 to 50 parts by mass of water, even more preferably 15 to 35 parts by mass of water, per 100 parts by mass of the water-soluble ethylenically unsaturated monomer. The amount of water herein refers to the total amount of the water contained in the reaction system and the water optionally used during the addition of the surface-crosslinking agent.

The reaction temperature during the surface-crosslinking reaction is preferably 50 to 250°C, more preferably 60 to 180°C, still more preferably 60 to 140°C, and even more preferably 70 to 120°C. The reaction time of the surface-crosslinking reaction is preferably 1 to 300 minutes, and more preferably 5 to 200 minutes.

<Drying Step>

After the reversed phase suspension polymerization is performed as described above, the method may include a drying step of adding external energy, such as heat, to remove the water, the hydrocarbon dispersion medium, and the like by distillation. To remove the water in the hydrous gel after the reversed phase suspension polymerization, the system in which the hydrous gel is dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. This is preferable in view of preventing deterioration of the resin, because the temperature within the system during drying is maintained at a temperature not higher than the azeotropic temperature with the hydrocarbon dispersion medium. Subsequently, the water and the hydrocarbon dispersion medium are distilled off to obtain water-absorbent resin particles. By controlling the treatment conditions for the drying step after the polymerization to adjust the amount of water to be removed, various kinds of performance of the resulting water-absorbent resin particles can be controlled.

In the drying step, the drying treatment by distillation may be performed under atmospheric pressure or reduced pressure. The drying treatment may also be performed in a stream of nitrogen or the like, in view of improving the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

When the surface-crosslinking step with a surface-crosslinking agent is performed after the polymerization of the monomer by the reversed phase suspension polymerization, the drying step by distillation is performed as described above, after the completion of the surface-crosslinking step. Alternatively, the surface-crosslinking step and the drying step may be performed simultaneously.

The water-absorbent resin particles obtained by the method of the present invention may contain additives suitable for its purpose. Examples of such additives include inorganic powders, surfactants, oxidizing agents, reducing agents, metal chelating agents, radical chain inhibitors, antioxidants, and anti-bacterial agents. For example, an inorganic powder may be used to further improve the flowability of the water-absorbent resin particles. The amount of the inorganic powder used is preferably in the range of 0.01 to 5 parts by mass, and more preferably 0.1 to 1 part by mass, per 100 parts by mass of the water-absorbent resin particles.

2. Water-Absorbent Resin Particles

Water-absorbent resin particles having the properties (A) to (C) shown below, for example, can be satisfactorily produced by adopting preferred conditions in view of improving the physiological saline retention capacity, the physiological saline absorption capacity under a load of 4.14 kPa, and the value of non-pressurization DW after 5 minutes, among those in the method for producing water-absorbent resin particles described above. For example, in the method for producing water-absorbent resin particles, the storage elastic modulus in each of the first- and second-stage polymerization steps preferably falls in the preferred ranges described above.

(A) A physiological saline retention capacity of 50 g/g or more and 80 g/g or less
(B) A physiological saline absorption capacity under a load of 4.14 kPa of 15 g/g or more and 40 g/g or less
(C) A value of non-pressurization DW after 5 minutes of 50 mL/g or more and 80 mL/g or less

The physiological saline retention capacity (A) of the water-absorbent resin particles of the present invention is preferably 50 g/g or more, while it is preferably 80 g/g or less, more preferably 70 g/g or less, and still more preferably 60 g/g or less. The physiological saline retention capacity (A) is preferably in the range of 50 to 70 g/g, and more preferably in the range of 50 to 60 g/g, for example.

The physiological saline absorption capacity under a load of 4.14 kPa (B) of the water-absorbent resin particles of the present invention is preferably 15 g/g or more, more preferably 17 g/g or more, and still more preferably 19 g/g or more, while it is preferably 29 g/g or less, more preferably 27 g/g or less, still more preferably 25 g/g or less, and even more preferably 23 g/g or less. The physiological saline absorption capacity under a load of 4.14 kPa (B) is preferably in the range of 15 to 29 g/g, and more preferably in the range of 17 to 25 g/g, for example.

The value of non-pressurization DW after 5 minutes (C) of the present invention is preferably 50 mL/g or more, more preferably 53 mL/g or more, still more preferably 55 mL/g or more, and particularly preferably 58 mL/g or more, while it is preferably 78 mL/g or less, more preferably 73 mL/g or less, and still more preferably 68 mL/g or less. The value of non-pressurization DW after 5 minutes (C) is preferably in the range of 50 to 78 mL/g, and more preferably in the range of 58 to 68 mL/g, for example.

The physiological saline retention capacity, physiological saline absorption capacity under load of 4.14 kPa, and value of non-pressurization DW after 5 minutes of the water-absorbent resin particles are each measured using the method described in the Examples.

The physiological saline absorption capacity of the water-absorbent resin particles is preferably in the following ranges. The physiological saline absorption capacity is preferably 30 g/g or more, 35 g/g or more, 40 g/g or more, 45 g/g or more, 50 g/g or more, 55 g/g or more, or 60 g/g or more, for example. The physiological saline absorption capacity is preferably 90 g/g or less, 85 g/g or less, 80 g/g or less, or 75 g/g or less, for example. The physiological saline absorption capacity is preferably 30 to 90 g/g, more preferably 40 to 80 g/g, and still more preferably 60 to 75 g/g. The method of measuring the physiological saline absorption capacity will be described in detail in the Examples below.

The water-absorbent resin particles have a water absorption rate with respect to physiological saline that is preferably in the following ranges. The water absorption rate is preferably 70 seconds or less, 60 seconds or less, 50 seconds or less, 45 seconds or less, or 40 seconds or less, for example. The water absorption rate is preferably 20 seconds or more, 25 seconds or more, or 30 seconds or more, for example. The water absorption rate is preferably 20 to 70 seconds, more preferably 25 to 60 seconds, and still more preferably 30 to 50 seconds. The water absorption rate may be the water absorption rate at room temperature (25 $\pm$ 2°C). The method of measuring the water absorption rate will be described in detail in the Examples below.

The water-absorbent resin particles of the present invention are formed of a crosslinked product of a polymer of a water-soluble ethylenically unsaturated monomer, that is, a crosslinked polymer having a structural unit derived from a water-soluble ethylenically unsaturated monomer.

The water-absorbent resin particles of the present invention are in a form in which fine particles (primary particles) are agglomerated (secondary particles). Examples of the shape of the primary particles include a substantially spherical shape, a crushed indefinite shape, and a flat shape. The water-absorbent resin particles of the present invention that are the secondary particles may have various shapes. Examples of the shape of the water-absorbent resin particles include a granular shape, a substantially spherical shape, a crushed indefinite shape, a flat shape, a fibrous shape, a flake shape, or a shape formed by agglomeration of such a resin. The water-absorbent resin particles preferably have, for example, a granular shape, a substantially spherical shape, a crushed indefinite shape, a fibrous shape, or a shape formed by agglomeration of such a resin.

The water-absorbent resin particles preferably have a median particle size of 200 $\mu$m or more, 250 $\mu$m or more, 280 $\mu$m or more, 300 $\mu$m or more, or 320 $\mu$m or more. From the same viewpoint, the median particle size is preferably 700 $\mu$m or less, 600 $\mu$m or less, 550 $\mu$m or less, 500 $\mu$m or less, 450 $\mu$m or less, or 400 $\mu$m or less. That is, the median particle size is preferably 200 to 700 $\mu$m, more preferably 200 to 600 $\mu$m, still more preferably 250 to 500 $\mu$m, even more preferably 300 to 450 $\mu$m, and still more preferably 320 to 400 $\mu$m.

The median particle size of the water-absorbent resin particles can be measured using JIS standard sieves; specifically, it is the value as measured by the method described in the Examples section.

3. Absorbent Material and Absorbent Article

The water-absorbent resin particles of the present invention constitute an absorbent material used for hygienic materials, such as sanitary items and disposable diapers, for example, and are suitably used for an absorbent article including the absorbent material.

The absorbent material of the present invention contains the water-absorbent resin particles of the present invention. The absorbent material may further contain hydrophilic fibers. Examples of the structure of the absorbent material include a sheet-like structure in which the water-absorbent resin particles are fixed on a nonwoven fabric or between a plurality of nonwoven fabrics; a mixed dispersion obtained by mixing the water-absorbent resin particles and hydrophilic fibers to give a homogeneous composition; a sandwich structure in which the water-absorbent resin particles are sandwiched between layered hydrophilic fibers; and a structure in which the water-absorbent resin particles and hydrophilic fibers are wrapped in a tissue. The absorbent material may also contain other components, for example, adhesive binders such as thermal bonding synthetic fibers, hot melt adhesives, and adhesive emulsions, for improving the shape retention properties of the absorbent material.

The water-absorbent resin particles in the absorbent material of the present invention have a basis weight of 50 g/m$^2$ or more and 400 g/m$^2$ or less. The basis weight is preferably 100 g/m$^2$ or more, more preferably 120 g/m$^2$ or more, and still more preferably 140 g/m$^2$ or more, while the basis weight is preferably 300 g/m$^2$ or less, more preferably 250 g/m$^2$ or less, and still more preferably 200 g/m$^2$ or less.

The hydrophilic fibers may be, for example, at least one selected from the group consisting of finely ground wood pulp, cotton, cotton linter, rayon, cellulose acetate, polyamides, polyesters, and polyolefins. Examples include cellulose fibers, such as cotton-like pulp made from wood, mechanical pulp, chemical pulp, and semi-chemical pulp; artificial cellulose fibers, such as rayon and acetate; and fibers made of synthetic resins, such as hydrophilized polyamides, polyesters, and polyolefins. The hydrophilic fibers typically have an average fiber length of 0.1 to 10 mm. Alternatively, the average fiber length may be 0.5 to 5 mm.

The hydrophilic fibers in the absorbent material of present invention have a basis weight of 50 g/m$^2$ or more and 800 g/m$^2$ or less. The basis weight is preferably 100 g/m$^2$ or more, more preferably 120 g/m$^2$ or more, and still more preferably 140 g/m$^2$ or more, while the basis weight is preferably 700 g/m$^2$ or less, more preferably 600 g/m$^2$ or less, and still more preferably 500 g/m$^2$ or less.

The content of the water-absorbent resin particles in the absorbent material is preferably 5 to 100% by mass, more preferably 10 to 95% by mass, still more preferably 20 to 90% by mass, and even more preferably 30 to 80% by mass.

It is possible to obtain the absorbent article of the present invention by holding the absorbent material obtained using the water-absorbent resin particles of the present invention between a liquid-permeable sheet (top sheet) that allows a liquid to pass through and a liquid-impermeable sheet (back sheet) that does not allow a liquid to pass through. The liquid-permeable sheet is positioned on the side that is brought into contact with the body, and the liquid-impermeable sheet is positioned opposite to the side that is brought into contact with the body.

Examples of the liquid-permeable sheet include porous synthetic resin sheets and nonwoven fabrics of the types such as air-through type, spunbond type, chemical bond type, and needle punch type, which are made of fibers of polyethylene, polypropylene, polyester, and the like. Examples of the liquid-impermeable sheet include synthetic resin films made of resins such as polyethylene, polypropylene, and polyvinyl chloride. The liquid-permeable sheet is preferably at least one selected from the group consisting of a thermally bonded nonwoven fabric, an air-through nonwoven fabric, a spunbond nonwoven fabric, and a spunbond/melt-blown/spunbond nonwoven fabric.

The liquid-permeable sheet has a basis weight of preferably 5 g/m$^2$ or more and 100 g/m$^2$ or less, more preferably 10 g/m$^2$ or more and 60 g/m$^2$ or less. The surface of the liquid-permeable sheet may be embossed or perforated in order to improve the liquid diffusibility. The embossing and perforating can be performed by known methods.

Examples of the liquid-impermeable sheet include a sheet made of a synthetic resin such as polyethylene, polypropylene, or polyvinyl chloride, a sheet made of a nonwoven fabric such as spunbond/melt-blown/spunbond (SMS) nonwoven fabric in which a water-resistant melt-blown nonwoven fabric is sandwiched between high-strength spunbond nonwoven fabrics, and a sheet made of a composite material of these synthetic resins and a nonwoven fabric (for example, a spunbond nonwoven fabric or a spunlace nonwoven fabric). As the liquid-impermeable sheet, a sheet made of a synthetic resin mainly containing a low-density polyethylene (LDPE) resin can also be used. The liquid-impermeable sheet may be, for example, a sheet made of a synthetic resin having a basis weight of 10 to 50 g/m$^2$.

The absorbent article preferably includes a laminate having an absorbent material containing the water-absorbent resin particles and core wraps sandwiching upper and lower portions of the absorbent material; a liquid-permeable sheet disposed on an upper surface of the laminate; and a liquid-impermeable sheet disposed on a surface of the laminate opposite to the liquid-permeable sheet side.

## 4. Additional Matters

The present specification includes at least the inventions as set forth in (1) to (14) below.

(1) A method for producing water-absorbent resin particles by subjecting a water-soluble ethylenically unsaturated monomer to reversed phase suspension polymerization using a radical polymerization initiator,

wherein the radical polymerization initiator comprises an azo compound as at least one radical polymerization initiator,
wherein the reversed phase suspension polymerization comprises two or more stages of polymerization steps,
wherein in a first-stage polymerization step,

(a) an internal-crosslinking agent is used in an amount of 0.01 mmol or more and 0.11 mmol or less per mole of the water-soluble ethylenically unsaturated monomer used in first-stage polymerization,
wherein in a second-stage polymerization step,

(b) an internal-crosslinking agent is used in an amount of 0 mmol or more and 0.042 mmol or less per mole of the water-soluble ethylenically unsaturated monomer used in second-stage polymerization, and
(c) the azo compound is used in an amount of 0.38 mmol or less per mole of the water-soluble ethylenically unsaturated monomer used in the second-stage polymerization, and

wherein the method comprises a surface-crosslinking step after the second- or subsequent-stage polymerization step.

(2) The method according to (1) above, wherein the amount of the internal-crosslinking agent used in the first-stage polymerization step is 0.01 to 0.11 mmol, 0.02 to 0.09 mmol, 0.03 to 0.075 mmol, or 0.04 to 0.06 mmol per mole of the water-soluble ethylenically unsaturated monomer used in the first-stage polymerization.

(3) The method according to (1) or (2) above, wherein the radical polymerization initiator used in the first-stage polymerization step comprises an azo compound as at least one radical polymerization initiator, and the amount of the azo compound used is 0 to 1.0 mmol, 0 to 0.50 mmol, 0.10 to 0.38 mmol, or 0.20 to 0.35 mmol per mole of the water-soluble ethylenically unsaturated monomer used in the first-stage polymerization.

(4) The method according to any one of (1) to (3) above, wherein the amount of the internal-crosslinking agent used in the second-stage polymerization step is 0 to 0.042 mmol, 0 to 0.038 mmol, 0 to 0.032 mmol, 0 to 0.030 mmol, or 0 to 0.025 mmol per mole of the water-soluble ethylenically unsaturated monomer used in the second-stage polymerization.

(5) The method according to any one of (1) to (4) above, wherein the amount of the azo compound used in the second-stage polymerization step is 0 to 0.38 mmol, 0 to 0.35 mmol, 0.05 to 0.35 mmol, or 0.10 to 0.35 mmol per mole of the water-soluble ethylenically unsaturated monomer used in the second-stage polymerization.

(6) The method according to any one of (1) to (5) above, wherein primary particles obtained in the first-stage polymerization step, when prepared as a gel swollen 40-fold with ion exchange water, have a storage elastic modulus of 10 Pa or more and 200 Pa or less, and
wherein secondary particles obtained in the second- or subsequent-stage polymerization step, when prepared as a gel swollen 40-fold with ion exchange water, have a storage elastic modulus of 5 Pa or more and 200 Pa or less.

(7) The method according to any one of (1) to (6) above, wherein the storage elastic modulus of the primary particles obtained in the first-stage polymerization step, when prepared as a gel swollen 40-fold with ion exchange water, is 15 to 150 Pa or 20 to 100 Pa.

(8) The method according to any one of (1) to (7) above, wherein the storage elastic modulus of the secondary particles obtained in the second-stage polymerization step, when prepared as a gel swollen 40-fold with ion exchange water, is 20 to 190 Pa, 20 to 170 Pa, 30 to 150 Pa, or 30 to 100 Pa.

(9) Water-absorbent resin particles, the water-absorbent resin particles being a crosslinked product of a polymer of a water-soluble ethylenically unsaturated monomer,
wherein the water-absorbent resin particles have the following properties (A) to (C):

(A) a physiological saline retention capacity of 50 g/g or more and 80 g/g or less;
(B) a physiological saline absorption capacity under a load of 4.14 kPa of 15 g/g or more and 40 g/g or less; and
(C) a value of non-pressurization DW after 5 minutes of 50 mL/g or more and 80 mL/g or less.

(10) The water-absorbent resin particles according to (9) above, wherein the physiological saline retention capacity of the water-absorbent resin particles is 50 to 70 g/g or 50 to 60 g/g.

(11) The water-absorbent resin particles according to (9) or (10) above, wherein the physiological saline absorption capacity under a load of 4.14 kPa of the water-absorbent resin particles is 15 to 29 g/g or 17 to 25 g/g.

(12) The water-absorbent resin particles according to any one of (9) to (11) above, wherein the value of non-pressurization DW after 5 minutes of the water-absorbent resin particles is 50 to 78 mL/g or 58 to 68 mL/g.

(13) The water-absorbent resin particles according to any one of (9) to (12) above, wherein the physiological saline absorption capacity of the water-absorbent resin particles is 40 to 80 g/g or 60 to 75 g/g.

(14) The water-absorbent resin particles according to any one of (9) to (13) above, wherein the water-absorbent resin particles have a water absorption rate with respect to physiological saline of 20 to 70 seconds, 25 to 60 seconds, or 30 to 50 seconds.

Examples

The present invention will be hereinafter described in detail with reference to examples and comparative examples. However, the present invention is not limited to the examples.

<Production of Water-Absorbent Resin Particles>

<Example 1>

[First-Stage Polymerization Step]

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 293 g of n-heptane was placed in this flask, and 0.782 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C.

In a 300 mL inner volume beaker, 92.0 g (1.03 mol) of an 80.5% by mass aqueous solution of acrylic acid was placed as a water-soluble ethylenically unsaturated monomer, and 110.1 g of a 28% by mass aqueous solution of sodium hydroxide was added dropwise, with external cooling, to achieve 75 mol% neutralization. Then, 0.092 g (0.339 mmol) of 2,2'-azobis(2-amidinopropane) dihydrochloride and 0.028 g (0.102 mmol) of potassium persulfate as water-soluble radical polymerization initiators, 0.0101 g (0.058 mmol) of ethylene glycol diglycidyl ether as an internal-crosslinking agent, and 37.2 g of ion exchange water were added and dissolved to prepare a first-stage aqueous monomer solution.

Then, the aqueous solution prepared as above was added to the separable flask and stirred for 10 minutes. Then, a surfactant solution prepared by heating and dissolving 0.828 g of sucrose stearate having an HLB of 3 (Ryoto sugar ester S-370 from Mitsubishi-Kagaku Foods Corporation) as a surfactant in 7.45 g of n-heptane was further added. The system was sufficiently purged with nitrogen while stirring with the agitator at a rotation speed of 500 rpm. Then, the flask was immersed in a water bath at 70°C and heated, and polymerization was performed for 60 minutes to give a first-stage polymerization slurry containing primary particles.

[Second-Stage Polymerization Step]

In a 500 mL inner volume beaker, 128.8 g (1.44 mol) of an 80.5% by mass aqueous solution of acrylic acid was placed as a water-soluble ethylenically unsaturated monomer, and 154.2 g of a 28% by mass aqueous solution of sodium hydroxide was added dropwise, with external cooling, to achieve 75 mol% neutralization. Then, 0.064 g (0.237 mmol) of 2,2'-azobis(2-amidinopropane) dihydrochloride and 0.039 g (0.143 mmol) of potassium persulfate as water-soluble radical polymerization initiators, and 6.49 g of ion exchange water were added and dissolved to prepare a second-stage aqueous monomer solution.

The separable flask system was cooled to 25°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes. A hydrous gel after the second-stage polymerization was obtained by the procedure described above.

<Surface-Crosslinking>

The flask was then immersed in an oil bath set at 125°C to remove 176.6 g of water out of the system by azeotropic distillation of the water and n-heptane, while refluxing the n-heptane. Then, 0.491 g of a 45% by mass aqueous solution of diethylenetriaminepentaacetic acid pentasodium salt and 2.58 g of a 3% by mass aqueous solution of sodium sulfite were added to the flask with stirring to give a second-stage polymerization slurry containing secondary particles.

The flask was then again immersed in an oil bath set at 125°C to further remove 80.5 g (257.1 g in total) of water out of the system by azeotropic distillation of the water and n-heptane, while refluxing the n-heptane. Then, 5.52 g (0.634 mmol) of a 2% by mass aqueous solution of ethylene glycol diglycidyl ether was added to the flask as a surface-crosslinking agent, and the mixture was maintained at 83°C for 2 hours.

Then, the mixture was dried by evaporating the n-heptane at 125°C to give a dried product. The dried product was passed through a sieve with a mesh size of 850 $\mu$m, and the resulting dried product was mixed with 0.2% by mass of amorphous silica (Tokusil NP-S from Oriental Silicas Corporation) to give 235.2 g of water-absorbent resin particles. The water-absorbent resin particles had a median particle size of 347 $\mu$m.

The water-absorbent resin particles of Example 1 had a physiological saline absorption capacity of 64 g/g and a water absorption rate of 35 seconds.

<Example 2>

223.4 g of water-absorbent resin particles were obtained as in Example 1, except that the amount of water removed out of the system by the second azeotropic distillation was changed to 90.2 g (266.8 g in total). The water-absorbent resin particles had a median particle size of 339 $\mu$m.
The water-absorbent resin particles of Example 2 had a physiological saline absorption capacity of 72 g/g and a water absorption rate of 33 seconds.

<Example 3>

226.0 g of water-absorbent resin particles were obtained as in Example 1, except that the amount of 2,2'-azobis(2-amidinopropane) dihydrochloride added to the second-stage aqueous monomer solution was changed to 0.129 g (0.475 mmol), the amount of ion exchange water added was changed to 6.42 g, and the amount of water removed out of the system by the second azeotropic distillation was changed to 80.3 g (256.9 g in total). The water-absorbent resin particles had a median particle size of 353 $\mu$m.
The water-absorbent resin particles of Example 3 had a physiological saline absorption capacity of 71 g/g and a water absorption rate of 39 seconds.

<Example 4>

224.3 g of water-absorbent resin particles were obtained as in Example 1, except that 0.0052 g (0.030 mmol) of ethylene glycol diglycidyl ether was added as an internal-crosslinking agent to the second-stage aqueous monomer solution, the amount of ion exchange water added to the second-stage aqueous monomer solution was changed to 6.48 g, and the amount of water removed out of the system by the second azeotropic distillation was changed to 83.9 g (260.6 g in total). The water-absorbent resin particles had a median particle size of 361 $\mu$m.
The water-absorbent resin particles of Example 4 had a physiological saline absorption capacity of 68 g/g and a water absorption rate of 39 seconds.

<Example 5>

225.7 g of water-absorbent resin particles were obtained as in Example 1, except that the amount of water removed out of the system by the second azeotropic distillation was changed to 89.9 g (266.5 g in total), and the amount of a 2% by mass aqueous solution of ethylene glycol diglycidyl ether added was changed to 8.28 g (0.951 mmol). The water-absorbent resin particles had a median particle size of 360 $\mu$m.
The water-absorbent resin particles of Example 5 had a physiological saline absorption capacity of 72 g/g and a water absorption rate of 40 seconds.

<Example 6>

221.5 g of water-absorbent resin particles were obtained as in Example 1, except that 0.0129 g (0.122 mmol) of sodium hypophosphite was added as a chain transfer agent to the second-stage aqueous monomer solution, the amount of ion exchange water added to the second-stage aqueous monomer solution was changed to 6.47 g, and the amount of water removed out of the system by the second azeotropic distillation was changed to 82.9 g (259.5 g in total). The water-absorbent resin particles had a median particle size of 369 $\mu$m.
The water-absorbent resin particles of Example 6 had a physiological saline absorption capacity of 64 g/g and a water absorption rate of 37 seconds.

<Example 7>

220.1 g of water-absorbent resin particles were obtained as in Example 1, except that the amount of ethylene glycol diglycidyl ether added to the first-stage aqueous monomer solution was changed to 0.0184 g (0.106 mmol), and the amount of water removed out of the system by the second azeotropic distillation was changed to 84.7 g (261.4 g in total). The water-absorbent resin particles had a median particle size of 423 $\mu$m.
The water-absorbent resin particles of Example 7 had a physiological saline absorption capacity of 67 g/g and a water absorption rate of 48 seconds.

<Example 8>

[First-Stage Polymerization Step]

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 294 g of n-heptane was placed in this flask, and 0.644 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C.

In a 300 mL inner volume beaker, 92.0 g (1.03 mol) of an 80.5% by mass aqueous solution of acrylic acid was placed as a water-soluble ethylenically unsaturated monomer, and 110.1 g of a 28% by mass aqueous solution of sodium hydroxide was added dropwise, with external cooling, to achieve 75 mol% neutralization. Then, 0.074 g (0.272 mmol) of potassium persulfate as a water-soluble radical polymerization initiator, 0.0046 g (0.026 mmol) of ethylene glycol diglycidyl ether as an internal-crosslinking agent, and 37.3 g of ion exchange water were added and dissolved to prepare a first-stage aqueous monomer solution.

Then, the aqueous solution prepared as above was added to the separable flask and stirred for 10 minutes. Then, a surfactant solution prepared by heating and dissolving 0.644 g of sucrose stearate having an HLB of 3 (Ryoto sugar ester S-370 from Mitsubishi-Kagaku Foods Corporation) as a surfactant in 5.8 g of n-heptane was further added. The system was sufficiently purged with nitrogen while stirring with the agitator at a rotation speed of 500 rpm. Then, the flask was immersed in a water bath at 70°C and heated, and polymerization was performed for 60 minutes to give a first-stage polymerization slurry containing primary particles.

[Second-Stage Polymerization Step]

In a 500 mL inner volume beaker, 128.8 g (1.44 mol) of an 80.5% by mass aqueous solution of acrylic acid was placed as a water-soluble ethylenically unsaturated monomer, and 154.2 g of a 28% by mass aqueous solution of sodium hydroxide was added dropwise, with external cooling, to achieve 75 mol% neutralization. Then, 0.064 g (0.237 mmol) of 2,2'-azobis(2-amidinopropane) dihydrochloride and 0.039 g (0.143 mmol) of potassium persulfate as water-soluble radical polymerization initiators, and 6.49 g of ion exchange water were added and dissolved to prepare a second-stage aqueous monomer solution.

The separable flask system was cooled to 25°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes. A hydrous gel after the second-stage polymerization was obtained by the procedure described above.

<Surface-Crosslinking>

The flask was then immersed in an oil bath set at 125°C to remove 176.6 g of water out of the system by azeotropic distillation of the water and n-heptane, while refluxing the n-heptane. Then, 0.491 g of a 45% by mass aqueous solution of diethylenetriaminepentaacetic acid pentasodium salt and 2.58 g of a 3% by mass aqueous solution of sodium sulfite were added to the flask with stirring to give a second-stage polymerization slurry containing secondary particles.

The flask was then again immersed in an oil bath set at 125°C to further remove 79.7 g (256.4 g in total) of water out of the system by azeotropic distillation of the water and n-heptane, while refluxing the n-heptane. Then, 5.52 g (0.634 mmol) of a 2% by mass aqueous solution of ethylene glycol diglycidyl ether was added to the flask as a surface-crosslinking agent, and the mixture was maintained at 83°C for 2 hours.

Then, the mixture was dried by evaporating the n-heptane at 125°C to give a dried product. The dried product was passed through a sieve with a mesh size of 850 $\mu$m, and the resulting dried product was mixed with 0.2% by mass of amorphous silica (Tokusil NP-S from Oriental Silicas Corporation) to give 214.9 g of water-absorbent resin particles. The water-absorbent resin particles had a median particle size of 372 $\mu$m.

The water-absorbent resin particles of Example 8 had a physiological saline absorption capacity of 73 g/g and a water absorption rate of 41 seconds.

<Comparative Example 1>

224.4 g of water-absorbent resin particles were obtained as in Example 8, except that 2,2'-azobis(2-amidinopropane) dihydrochloride was not added to the second-stage aqueous monomer solution; the amount of potassium persulfate added to the second-stage aqueous monomer solution was changed to 0.090 g (0.334 mmol); 0.0116 g (0.067 mmol) of ethylene glycol diglycidyl ether was added to the second-stage aqueous monomer solution; and the amount of water removed out of the system by the second azeotropic distillation was changed to 95.4 g (272.0 g in total). The water-absorbent resin particles had a median particle size of 347 μm.

The water-absorbent resin particles of Comparative Example 1 had a physiological saline absorption capacity of 67 g/g and a water absorption rate of 39 seconds.

<Comparative Example 2>

[First-Stage Polymerization Step]

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 265 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C.

In a 300 mL inner volume beaker, 92.0 g (1.03 mol) of an 80.5% by mass aqueous solution of acrylic acid was placed as a water-soluble ethylenically unsaturated monomer, and 110.1 g of a 28% by mass aqueous solution of sodium hydroxide was added dropwise, with external cooling, to achieve 75 mol% neutralization. Then, 0.092 g of hydroxylethylcellulose (HECAW-15F from Sumitomo Seika Chemicals Co., Ltd.) as a thickener, 0.092 g (0.339 mmol) of 2,2'-azobis(2-amidinopropane) dihydrochloride and 0.028 g (0.102 mmol) of potassium persulfate as water-soluble radical polymerization initiators, 0.0046 g (0.026 mmol) of ethylene glycol diglycidyl ether as an internal-crosslinking agent, and 14.3 g of ion exchange water were added and dissolved to prepare a first-stage aqueous monomer solution.

Then, the aqueous solution prepared as above was added to the separable flask and stirred for 10 minutes. Then, a surfactant solution prepared by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the agitator at a rotation speed of 600 rpm. Then, the flask was immersed in a water bath at 70°C and heated, and polymerization was performed for 60 minutes to give a first-stage polymerization slurry containing primary particles.

[Second-Stage Polymerization Step]

In a 500 mL inner volume beaker, 128.8 g (1.44 mol) of an 80.5% by mass aqueous solution of acrylic acid was placed as a water-soluble ethylenically unsaturated monomer, and 154.2 g of a 28% by mass aqueous solution of sodium hydroxide was added dropwise, with external cooling, to achieve 75 mol% neutralization. Then, 0.129 g (0.475 mmol) of 2,2'-azobis(2-amidinopropane) dihydrochloride and 0.039 g (0.143 mmol) of potassium persulfate as water-soluble radical polymerization initiators, 0.0116 g (0.067 mmol) of ethylene glycol diglycidyl ether as an internal-crosslinking agent, and 6.41 g of ion exchange water were added and dissolved to prepare a second-stage aqueous monomer solution.

The separable flask system was cooled to 25°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes. A hydrous gel after the second-stage polymerization was obtained by the procedure described above.

<Surface-Crosslinking>

The flask was then immersed in an oil bath set at 125°C to remove 176.6 g of water out of the system by azeotropic distillation of the water and n-heptane, while refluxing the n-heptane. Then, 0.393 g of a 45% by mass aqueous solution of diethylenetriaminepentaacetic acid pentasodium salt and 4.42 g of a 3% by mass aqueous solution of sodium sulfite were added to the flask with stirring to give a second-stage polymerization slurry containing secondary particles.

The flask was then again immersed in an oil bath set at 125°C to further remove 49.3 g (226.0 g in total) of water out of the system by azeotropic distillation of the water and n-heptane, while refluxing the n-heptane. Then, 4.42 g (0.507 mmol) of a 2% by mass aqueous solution of ethylene glycol diglycidyl ether was added to the flask as a surface-crosslinking agent, and the mixture was maintained at 83°C for 2 hours.

Then, the mixture was dried by evaporating the n-heptane at 125°C to give a dried product. The dried product was passed through a sieve with a mesh size of 850 μm, and the resulting dried product was mixed with 0.2% by mass of amorphous silica (Tokusil NP-S from Oriental Silicas Corporation) to give 229.4 g of water-absorbent resin particles. The water-absorbent resin particles had a median particle size of 352 μm.

The water-absorbent resin particles of Comparative Example 2 had a physiological saline absorption capacity of 66 g/g and a water absorption rate of 36 seconds.

[Measurement of Storage Elastic Modulus of Primary Particles (Gel Swollen 40-Fold with Ion Exchange Water)]

A first-stage polymerization slurry was obtained in the production of water-absorbent resin particles. Then, the reaction mixture was heated in an oil bath set at 125°C to remove water out of the system by azeotropic distillation of the water and n-heptane, while refluxing the n-heptane, until the temperature in the system reached 88°C. Then, the mixture was dried by evaporating the n-heptane at 125°C to give a dried product. The dried product was passed through a sieve with a mesh size of 850 $\mu$m, and the resulting dried product was mixed with 0.2% by mass of amorphous silica (Tokusil NP-S from Oriental Silicas Corporation) to give primary particles.

Next, the primary particles were swollen 40-fold with ion exchange water to prepare a 40-fold swollen gel. Specifically, 195.0 g of ion exchange water was first weighed into a 500 mL beaker. Next, the beaker containing the ion exchange water was placed in a jar tester (MJS-8S) from MIYAMOTO CORPORATION. A stirring blade having a stainless steel flat blade (7 cm in horizontal length $\times$ 2.5 cm in vertical length $\times$ 0.1 cm in thickness) was connected to the beaker, placed at a height of 0.5 cm from the beaker bottom, and rotated at 500 rpm. Next, 5.0 g of the primary particles were added into the stirred beaker so as to prevent formation of unswollen lumps. One minute after the addition, the rotation speed was changed to 300 rpm, and stirring was continued for 1 hour after the addition to prepare a 40-fold swollen gel. The 40-fold swollen gel was transferred into a centrifuge tube and degassed by centrifugation at a rotation speed of 2000 rpm for 10 minutes in a centrifuge (product number H-36 from Kokusan Centrifuge Co., Ltd.) to give a measurement sample.

The storage elastic modulus was measured by creep measurement using a stress-controlled rheometer (product number AR2000ex from TA Instruments Ltd). The measurement temperature was 25°C, and an aluminum parallel plate with a diameter of 60 mm (product number 513600.901 from TA Instruments Ltd.) was used as the jig. The shear stress was measured at 0.7 Pa.

The measurement sample was weighed out using a 15 mL dispensing spoon, and placed on the sample table so as not to introduce air bubbles into the measurement sample. Then, the jig was lowered so that the lower portion of the jig was filled with the measurement sample, and the gap between the jig and the sample table was adjusted to 4000 $\mu$m. Any measurement sample lying outside the jig was removed. Creep measurement was performed, and the storage elastic modulus was calculated by the Jeffreys underdamped ringing analysis. The results are shown in Table 1.

[Measurement of Storage Elastic Modulus of Secondary Particles (Gel Swollen 40-Fold with Ion Exchange Water)]

A second-stage polymerization slurry was obtained in the production of water-absorbent resin particles. Then, the reaction mixture was heated in an oil bath set at 125°C to remove water out of the system by azeotropic distillation of the water and n-heptane, while refluxing the n-heptane, until the temperature in the system reached 88°C. Then, the mixture was dried by evaporating the n-heptane at 125°C to give a dried product. The dried product was passed through a sieve with a mesh size of 850 $\mu$m, and the resulting dried product was mixed with 0.2% by mass of amorphous silica (Tokusil NP-S from Oriental Silicas Corporation) to give secondary particles. Thereafter, the storage elastic modulus was measured using the same procedure as in the method of measuring the storage elastic modulus of the primary particles. The results are shown in Table 1.

<Physiological Saline Retention Capacity>

2.0 g of the water-absorbent resin particles were weighed into a cotton bag (Cottonbroad No. 60, 100 mm in width $\times$ 200 mm in length), and the cotton bag was placed in a 500 mL beaker. 500 g of a 0.9% by mass aqueous solution of sodium chloride (physiological saline) was poured at once into the cotton bag containing the water-absorbent resin particles so as to prevent formation of unswollen lumps, then the top of the cotton bag was closed with a rubber band, and the cotton bag was allowed to stand for 30 minutes to cause the water-absorbent resin particles to swell. After 30 minutes, the cotton bag was dehydrated for 1 minute using a dehydrator (product number: H-122 from Kokusan Co., Ltd.) set at a centrifugal force of 167 G, and a mass Wa (g) of the cotton bag containing the swollen gel after dehydration was measured. The same procedure was performed without adding the water-absorbent resin particles, and an empty mass Wb (g) of the cotton bag upon wetting was measured. The physiological saline retention capacity was calculated using the following equation. The results are shown in Table 1.

$$\text{Physiological saline retention capacity (g/g)} = [\text{Wa} - \text{Wb}]/2.0$$

[Value of Non-Pressurization DW after 5 Minutes]

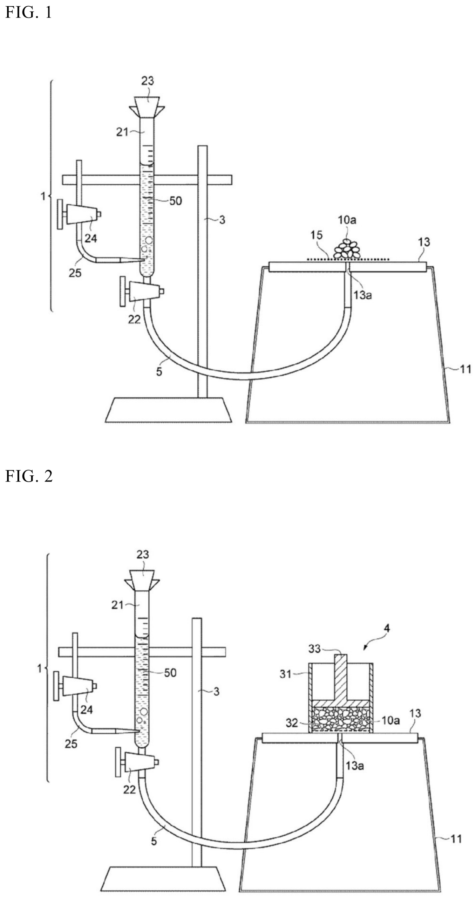

The value of non-pressurization DW after 5 minutes of the water-absorbent resin particles was measured using the measurement apparatus shown in Fig. 1. The value of non-pressurization DW after 5 minutes was measured five times for one type of water-absorbent resin particles, and the average value of three measured values excluding the minimum and maximum values was obtained.

The measurement apparatus includes a burette unit 1, a conduit 5, a measurement table 13, a nylon mesh sheet 15, a stand 11, and a clamp 3. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through-hole 13a with a diameter of 2 mm formed in the center portion thereof, and is supported by the variable-height stand 11. The through-hole 13a of the measurement table 13 and the cock 22 of the burette unit 1 are connected through the conduit 5. The conduit 5 has an inner diameter of 6 mm.

First, the cock 22 and the cock 24 of the burette unit 1 were closed, and 0.9% by mass saline 50 adjusted to 25°C was placed in the burette tube 21 through the upper opening of the burette tube 21. The saline concentration of 0.9% by mass is the concentration based on the mass of the saline. The opening of the burette tube 21 was sealed with the rubber stopper 23, and then the cock 22 and the cock 24 were opened. The conduit 5 was filled with the 0.9% by mass saline 50 to prevent air bubbles from entering. The height of the measurement table 13 was adjusted so that the height of the surface of the 0.9% by mass saline reached into the through-hole 13a was equal to the height of the upper surface of the measurement table 13. After the adjustment, the height of the surface of the 0.9% by mass saline 50 in the burette tube 21 was read on the graduations of the burette tube 21, and this position was determined as a zero point (reading at 0 seconds).

The nylon mesh sheet 15 (100 mm $\times$ 100 mm, 250 mesh, thickness about 50 $\mu$m) was laid in the vicinity of the through-hole 13a on the measurement table 13, and a cylinder having an inner diameter of 30 mm and a height of 20 mm was placed on the center portion of the nylon mesh sheet. 1.00 g of water-absorbent resin particles 10a were uniformly dispersed in this cylinder. Then, the cylinder was carefully removed to obtain a sample in which the water-absorbent resin particles 10a were dispersed in a circle shape in the center portion of the nylon mesh sheet 15. Subsequently, the nylon mesh sheet 15 on which the water-absorbent resin particles 10a were placed was moved so rapidly that the water-absorbent resin particles 10a did not dissipate, so that the center of the nylon mesh sheet 15 was at the position of the through-hole 13a, and then the measurement was started. The time when air bubbles were first introduced through the air inlet pipe 25 into the burette tube 21 was defined as the start of absorption (0 seconds).

An amount of reduction in the 0.9% by mass saline 50 in the burette tube 21 (that is, the amount of the 0.9% by mass saline absorbed by the water-absorbent resin particles 10a) was sequentially read by units of 0.1 mL, and a reduction in weight Wc (mL) of the 0.9% by mass saline 50 was read 5 minutes after the start of absorption by the water-absorbent resin particles 10a. The value of non-pressurization DW after 5 minutes was determined from Wc using the following equation. The non-pressurization DW is the water absorption capacity per 1.00 g of the water-absorbent resin particles 10a. The results are shown in Table 1.

$$\text{Value of non-pressurization DW after 5 minutes (mL/g)} = Wc/1.00$$

<Water Absorption Capacity under a Load of 4.14 kPa>

The water absorption capacity with respect to physiological saline under a load of 4.14 kPa (water absorption capacity under a load of 4.14 kPa) of the water-absorbent resin particles was measured using the apparatus schematically shown in Fig. 2. The water absorption capacity under load was measured twice for one type of water-absorbent resin particles, and the average value of the measured values was obtained. The apparatus shown in Fig. 2 includes a burette unit 1, a clamp 3, a conduit 5, a stand 11, a measurement table 13, and a measurement unit 4 placed on the measurement table 13. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through-hole 13a with a diameter of 2 mm formed in the center portion thereof, and is supported by the variable-height stand 11. The through-hole 13a of the measurement table 13 and the cock 22 of the burette unit 1 are connected through the conduit 5. The conduit 5 has an inner diameter of 6 mm.

The measurement unit 4 includes a cylinder 31 made of acrylic resin, a polyamide mesh 32 bonded to one opening of the cylinder 31, and a weight 33 vertically movable within the cylinder 31. The cylinder 31 is placed on the measurement table 13 with the polyamide mesh 32 therebetween. The cylinder 31 has an inner diameter of 20 mm. The polyamide mesh 32 has a mesh size of 75 $\mu$m (200 mesh). The weight 33 has a diameter of 19 mm and a mass of 119.6 g and, as described later, can apply a load of 4.14 kPa to water-absorbent resin particles 10a uniformly disposed on the polyamide mesh 32.

First, the cock 22 and the cock 24 of the burette unit 1 were closed, and 0.9% by mass physiological saline adjusted to 25°C was placed in the burette tube 21 through the upper opening of the burette tube 21. Next, the upper opening of the burette tube 21 was sealed with the rubber stopper 23, and then the cock 22 and the cock 24 were opened. The conduit 5 was filled with the 0.9% by mass saline 50 so as to prevent air bubbles from entering. The height of the measurement table 13 was adjusted so that the height of the surface of the 0.9% by mass saline reached into the through-hole 13a was equal to the height of the upper surface of the measurement table 13. After the adjustment, the height of the surface of the 0.9% by mass saline 50 in the burette tube 21 was read on the graduations of the burette tube 21, and this position was determined as a zero point (reading at 0 seconds).

In the measurement unit 4, 0.10 g of the water-absorbent resin particles 10a were uniformly disposed on the polyamide mesh 32 in the cylinder 31, the weight 33 was disposed on the water-absorbent resin particles 10a, and the cylinder 31 was placed such that the center portion thereof aligned with the conduit opening at the center portion of the measurement table 13. An amount of reduction Wd (mL) in the physiological saline in the burette tube 21 at 60 minutes after the beginning of absorption of the physiological saline by the water-absorbent resin particles 10a through the conduit 5 (that is, the amount of the physiological saline absorbed by the water-absorbent resin particles 10a) was read, and the physiological saline absorption capacity under a load of 4.14 kPa of the water-absorbent resin particles 10a was calculated using the following equation. The results are shown in Table 1.

Physiological saline absorption capacity under a load of 4.14 kPa (g/g) = Wd (mL) $\times$ 1.0028 [g/mL] (specific gravity of physiological saline)/0.10

[Physiological Saline Absorption Capacity]

500 g of 0.9% physiological saline was weighed into a 500 mL beaker. Next, 2.0 g of the water-absorbent resin particles were dispersed in the physiological saline while stirring at 600 rpm using a magnetic stirrer bar (8 mm $\phi$ $\times$ 30 mm in length, no ring) so as to prevent formation of unswollen lumps. The particles were left under stirring for 60 minutes and sufficiently swollen to give a dispersion containing a swollen gel. Subsequently, the mass We [g] of a standard sieve with a mesh size of 75 $\mu$m was measured, and then the dispersion described above was passed through this standard sieve. Then, excess water was removed by inclining the sieve at an inclination angle of about 30 degrees with respect to the horizontal direction, and leaving the sieve in this state for 30 minutes. The mass Wf [g] of the sieve on which the swollen gel remained was measured, and the physiological saline absorption capacity Wg [g/g] was determined using the following equation:

$$\text{Physiological saline absorption capacity Wg [g/g]} = (\text{Wf} - \text{We})/2.0$$

[Median Particle Size]

50 g of the water-absorbent resin particles were used for median particle size measurement. JIS standard sieves having mesh sizes of 710 $\mu$m, 600 $\mu$m, 500 $\mu$m, 425 $\mu$m, 300 $\mu$m, 250 $\mu$m, and 150 $\mu$m, and a receiving tray were combined in that order from the top. The water-absorbent resin particles were placed on the top sieve of the combined sieves, and classified by shaking for 10 minutes using a Ro-Tap shaker. After the classification, the particle size distribution was determined by calculating the mass of the water-absorbent resin particles remaining on each sieve as the mass percentage relative to the total mass. With regard to this particle size distribution, the mass percentage of the water-absorbent resin particles remaining on each sieve was integrated in descending order of particle size. In this manner, the relationship between the sieve mesh size and the cumulative value of the mass percentage of the water-absorbent resin particles remaining on each sieve was plotted on logarithmic probability paper. The plots on the probability paper were connected with straight lines, and a particle size equivalent to a 50% by mass cumulative mass percentage was determined as the median particle size.

<Water Absorption Rate (Vortex Method)>

The water absorption rate with respect to physiological saline of the water-absorbent resin particles was measured by the following procedure based on the Vortex method. First, 50 $\pm$ 0.1 g of physiological saline adjusted to a temperature of 25 $\pm$ 0.2°C in a thermostat was weighed into a 100 mL beaker. Next, the physiological saline was stirred with a magnetic stirrer bar (8 mm $\phi$ $\times$ 30 mm, no ring) at 600 rpm to form a vortex. 2.0 $\pm$ 0.002 g of the water-absorbent resin

**EP 4 696 719 A1**

particles were added at once into the physiological saline. The time [seconds] from the addition of the water-absorbent resin particles to the point in time at which the vortex on the liquid surface disappeared was measured. The measured time was recorded as the water absorption rate of the water-absorbent resin particles.

[Table 1]

| | Storage Elastic Modulus of Primary Particles | Storage Elastic Modulus of Secondary Particles | Physiological Saline Retention Capacity | Value of Non-Pressurization DW After 5 Minutes | Physiological Saline Absorption Capacity Under a Load of 4.14 kPa |
|---|---|---|---|---|---|
| | [Pa] | [Pa] | [g/g] | [mL/g] | [g/g] |
| Ex.1 | 38 | 98 | 51 | 53 | 21 |
| Ex.2 | 38 | 98 | 55 | 58 | 17 |
| Ex.3 | 38 | 63 | 53 | 60 | 19 |
| Ex.4 | 38 | 163 | 50 | 64 | 17 |
| Ex.5 | 38 | 98 | 52 | 62 | 20 |
| Ex.6 | 38 | 37 | 53 | 63 | 21 |
| Ex.7 | 112 | 186 | 51 | 67 | 15 |
| Ex.8 | 162 | 112 | 53 | 55 | 16 |
| Comp.Ex.1 | 162 | 500 | 53 | 39 | 8 |
| Comp.Ex.2 | 3 | 142 | 50 | 43 | 22 |

Reference Signs List

1:      burette unit
3:      clamp
4:      measurement unit
5:      conduit
10a:   water-absorbent resin particles
11:     stand
13:     measurement table
13a:   through-hole
15:     nylon mesh sheet
21:     burette tube
22:     cock
23:     rubber stopper
24:     cock
25:     air inlet tube
31:     cylinder
32:     polyamide mesh
33:     weight
50:     saline

**Claims**

1.  A method for producing water-absorbent resin particles by subjecting a water-soluble ethylenically unsaturated monomer to reversed phase suspension polymerization using a radical polymerization initiator,

    wherein the radical polymerization initiator comprises an azo compound as at least one radical polymerization initiator,

wherein the reversed phase suspension polymerization comprises two or more stages of polymerization steps, wherein in a first-stage polymerization step,

> (a) an internal-crosslinking agent is used in an amount of 0.01 mmol or more and 0.11 mmol or less per mole of the water-soluble ethylenically unsaturated monomer used in first-stage polymerization,
> wherein in a second-stage polymerization step,
> (b) an internal-crosslinking agent is used in an amount of 0 mmol or more and 0.042 mmol or less per mole of the water-soluble ethylenically unsaturated monomer used in second-stage polymerization, and
> (c) the azo compound is used in an amount of 0.38 mmol or less per mole of the water-soluble ethylenically unsaturated monomer used in the second-stage polymerization, and

wherein the method comprises a surface-crosslinking step after the second- or subsequent-stage polymerization step.

2. The method according to claim 1, wherein primary particles obtained in the first-stage polymerization step, when prepared as a gel swollen 40-fold with ion exchange water, have a storage elastic modulus of 10 Pa or more and 200 Pa or less, and
wherein secondary particles obtained in the second- or subsequent-stage polymerization step, when prepared as a gel swollen 40-fold with ion exchange water, have a storage elastic modulus of 5 Pa or more and 200 Pa or less.

3. The method according to claim 1 or 2, wherein the water-absorbent resin particles have the following properties (A) to (C):

> (A) a physiological saline retention capacity of 50 g/g or more and 80 g/g or less,
> (B) a physiological saline absorption capacity under a load of 4.14 kPa of 15 g/g or more and 40 g/g or less, and
> (C) a value of non-pressurization DW after 5 minutes of 50 mL/g or more and 80 mL/g or less.

4. Water-absorbent resin particles, the water-absorbent resin particles being a crosslinked product of a polymer of a water-soluble ethylenically unsaturated monomer,
wherein the water-absorbent resin particles have the following properties (A) to (C):

> (A) a physiological saline retention capacity of 50 *g/g* or more and 80 g/g or less,
> (B) a physiological saline absorption capacity under a load of 4.14 kPa of 15 g/g or more and 40 g/g or less, and
> (C) a value of non-pressurization DW after 5 minutes of 50 mL/g or more and 80 mL/g or less.

5. An absorbent material comprising the water-absorbent resin particles according to claim 4.

6. An absorbent article comprising the absorbent material according to claim 5.

FIG. 1

FIG. 2

## INTERNATIONAL SEARCH REPORT

| International application No. |
|---|
| **PCT/JP2024/014596** |

### A. CLASSIFICATION OF SUBJECT MATTER

*C08F 2/20*(2006.01)i; *C08F 20/06*(2006.01)i; *C08J 3/24*(2006.01)i; *C08L 33/02*(2006.01)i
FI:   C08F2/20; C08L33/02; C08J3/24 Z CEY; C08F20/06

According to International Patent Classification (IPC) or to both national classification and IPC

### B. FIELDS SEARCHED

Minimum documentation searched (classification system followed by classification symbols)

C08F2/00-2/60; 6/00-246/00; 301/00; C08J3/00-3/28; 99/00; C08L1/00-101/14; C08K3/00-13/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2020-121297 A (SUMITOMO SEIKA CHEMICALS CO., LTD.) 13 August 2020 (2020-08-13)<br>claims 1-5, paragraphs [0030], [0036], [0045], [0050], [0095]-[0101], [0103], [0120], [0122], [0127], table 1, example 3 | 1-6 |
| X | JP 7129490 B2 (SUMITOMO SEIKA CHEMICALS CO., LTD.) 01 September 2022 (2022-09-01)<br>claims 1-5, paragraphs [0026], [0032], [0037], [0044], [0101]-[0107], [0109]-[0110], [0118], [0138], table 1, example 4 | 1-6 |
| A | WO 2016/006134 A1 (SUMITOMO SEIKA CHEMICALS CO., LTD.) 14 January 2016 (2016-01-14)<br>entire text | 1-6 |
| A | WO 2020/184393 A1 (SUMITOMO SEIKA CHEMICALS CO., LTD.) 17 September 2020 (2020-09-17)<br>entire text | 1-6 |

☑ Further documents are listed in the continuation of Box C.　　☑ See patent family annex.

| | |
|---|---|
| *　Special categories of cited documents:<br>"A"　document defining the general state of the art which is not considered to be of particular relevance<br>"D"　document cited by the applicant in the international application<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search | Date of mailing of the international search report |
|---|---|
| **20 June 2024** | **02 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/014596**

| C. | DOCUMENTS CONSIDERED TO BE RELEVANT | |
|---|---|---|
| Category* | Citation of document, with indication, where appropriate, of the relevant passages | Relevant to claim No. |
| A | WO 2015/163438 A1 (NIPPON SHOKUBAI CO., LTD.) 29 October 2015 (2015-10-29) entire text | 1-6 |

Form PCT/ISA/210 (second sheet) (July 2022)

**INTERNATIONAL SEARCH REPORT**
Information on patent family members

International application No.

**PCT/JP2024/014596**

| Patent document cited in search report | | | Publication date (day/month/year) | Patent family member(s) | | | Publication date (day/month/year) |
|---|---|---|---|---|---|---|---|
| JP | 2020-121297 | A | 13 August 2020 | JP | 6681495 | B1 | |
| | | | | US | 2022/0023115 | A1 | |
| | | | | claims 1-4, paragraphs [0045], [0052], [0061], [0066], [0113]-[0119], [0121], [0138], [0140], table 1, example 3 | | | |
| | | | | WO | 2020/122218 | A1 | |
| | | | | EP | 3896120 | A1 | |
| | | | | CN | 113195598 | A | |
| | | | | KR | 10-2021-0101253 | A | |
| JP | 7129490 | B2 | 01 September 2022 | US | 2022/0055014 | A1 | |
| | | | | claims 1-5, paragraphs [0030], [0036], [0041], [0048], [0108]-[0114], [0116]-[0117], [0125], table 1, example 4 | | | |
| | | | | EP | 3896118 | A1 | |
| | | | | CN | 113195599 | A | |
| | | | | KR | 10-2021-0101252 | A | |
| | | | | WO | 2020/122219 | A1 | |
| WO | 2016/006134 | A1 | 14 January 2016 | JP | 2016-28118 | A | |
| | | | | US | 2016/0367717 | A1 | |
| | | | | entire text | | | |
| | | | | JP | 5766344 | B1 | |
| | | | | EP | 2998325 | A1 | |
| | | | | KR | 10-2016-0017650 | A | |
| | | | | CN | 105408365 | A | |
| WO | 2020/184393 | A1 | 17 September 2020 | US | 2022/0152581 | A1 | |
| | | | | entire text | | | |
| | | | | EP | 3936529 | A1 | |
| | | | | CN | 113544161 | A | |
| | | | | KR | 10-2021-0137066 | A | |
| WO | 2015/163438 | A1 | 29 October 2015 | US | 2017/0044281 | A1 | |
| | | | | entire text | | | |
| | | | | EP | 3135700 | A1 | |
| | | | | CN | 106255708 | A | |
| | | | | KR | 10-2016-0149227 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H3227301 A